(19) Europäisches Patentamt
European Patent Office
Office européen des brevets

(11) Veröffentlichungsnummer: **0 255 126 B1**

(12) **EUROPÄISCHE PATENTSCHRIFT**

(45) Veröffentlichungstag der Patentschrift: **14.10.92**

(51) Int. Cl.[5]: **C07C 219/06**, C07C 323/00, C07D 295/084, C07D 241/04

(21) Anmeldenummer: **87111007.8**

(22) Anmeldetag: **29.07.87**

Die Akte enthält technische Angaben, die nach dem Eingang der Anmeldung eingereicht wurden und die nicht in dieser Patentschrift enthalten sind.

# (54) 2-Acyloxypropylamin-Derivate, Verfahren zur Herstellung und diese enthaltende Arzneimittel.

(30) Priorität: **30.07.86 DE 3625738**

(43) Veröffentlichungstag der Anmeldung:
**03.02.88 Patentblatt 88/05**

(45) Bekanntmachung des Hinweises auf die Patenterteilung:
**14.10.92 Patentblatt 92/42**

(84) Benannte Vertragsstaaten:
**AT BE CH DE ES FR GB GR IT LI LU NL SE**

(56) Entgegenhaltungen:
**DE-A- 2 820 892**
**FR-A- 2 315 919**
**GB-A- 1 448 448**
**GB-A- 2 172 284**

**CHEMICAL ABSTRACTS, Band 66, Nr. 13, 27. März 1967, Seite 5175, Spalte 1, Zusammenfassungsnr. 54966a, Columbus, Ohio, US; S. MAMEDOV et al.: "Ethers of glycols and their derivates. C VIII. Synthesis of alkoxymethyl ethers of 1-hexoxy-3-diethylamino 2 propanol"**

(73) Patentinhaber: **GÖDECKE AKTIENGESELLSCHAFT**
**Salzufer 16**
**W-1000 Berlin 10(DE)**

(72) Erfinder: **Trostmann, Uwe, Dr.**
**Mühlenweg 14**
**W-7806 March-Hugstetten(DE)**
Erfinder: **Mannhardt, Karl, Dr.**
**Graf-Toerringstrasse 2**
**W-8031 Steinebach a. Wörthsee(DE)**
Erfinder: **Marmé, Dieter, Prof. Dr.**
**Kaschnitzweg 25**
**W-7800 Freiburg(DE)**
Erfinder: **Schächtele, Christoph, Dr.**
**Spechtweg 31**
**W-7800 Freiburg(DE)**
Erfinder: **Rudolph, Claus, Dr.**
**Gewerbestrasse 22**
**W-7803 Gundelfingen(DE)**

EP 0 255 126 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

HOUBEN-WEYL "Methoden der Organischen Chemie", Band VIII, 1952, Georg-Thieme Verlag, Stuttgart, DE

CHEMICAL ABSTRACTS, Band 90, Nr. 19, 7. Mai 1979, Seite 554, Spalte 2, Zusammenfassungsnr. 151539s, Columbus, Ohio, US; KORSHUMOV et al.: "Esters of alpha, beta unsaturated acid with functional groups in the alkoxy radical. XXII. 1(tertamino)-3-(alkylthio)isopropyl acrylates and metacylates"

## Beschreibung

Die Erfindung betrifft neue 2-Acyloxypropylamin-Derivate der allgemeinen Formel I

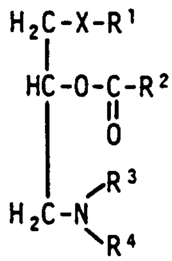

(I)

in welcher

- $R^1$ eine geradkettige oder verzweigte, gesättigte oder ungesättigte Alkylgruppe mit bis zu 20 Kohlenstoffatomen, darstellt,
- $R^2$ eine geradkettige oder verzweigte Alkylgruppe mit bis zu 4 Kohlenstoffatomen bedeutet,
- $R^3$ und $R^4$ die gleich oder verschieden sein können, Wasserstoff oder eine gegebenenfalls phenylsubstituierte geradkettige oder verzweigte Alkylgruppe mit bis zu 14 Kohlenstoffatomen oder gemeinsam einen unsubstituierten oder $C_1$ bis $C_4$ alkylsubstituierten Piperidin- oder Piperazinring bilden und
- X Sauerstoff oder Schwefel bedeuten,

mit Ausnahme von 3-Diethylamino-1-hexyloxy-2-propylacetat,
sowie deren pharmakologisch unbedenkliche Salze.

Bevorzugt werden 2-Acyloxypropylamin-Derivate der allgemeinen Formel I, in welcher
$R^1$ eine Propyl- , Octadecyl-, Octadecenyl-, Tetradecyl-, Hexadecyl-, Eicosanoyl- oder Isobutylgruppe,
$R^2$ eine Methyl- oder Isopropylgruppe,
$R^3$ und $R^4$, die gleich oder verschieden sein können, Wasserstoff, Methyl-, Ethyl-, Propyl- oder Benzylgruppen oder gemeinsam eine Piperidin- oder 2-Methylpiperazingruppe und
X Sauerstoff oder Schwefel bedeuten.

Besonders bevorzugt wird die Verbindung (±)-3-Dimethylamino-1-octadecyloxy-2-propylacetat und deren Salze.

Die Erfindung betrifft weiterhin ein Verfahren zur Herstellung von 2-Acyloxypropylamin-Derivaten der allgemeinen Formel I das dadurch gekennzeichnet ist, daß man

1.) in an sich bekannter Weise eine Verbindung der allgemeinen Formel II

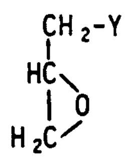

(II)

in welcher Y eine gute Abgangsgruppe, wie z.B. eine Tosylgruppe oder ein Halogenatom, bedeutet, mit einer Verbindung der allgemeinen Formel III,

$HX-R^1$ (III)

in der $R^1$ und X die oben angegebene Bedeutung haben, entweder

a) für den Fall, daß X Sauerstoff ist, in einem organisch/wäßrigen Zweiphasensystem umsetzt zu Verbindungen der allgemeinen Formel IV

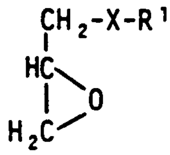

(IV)

in denen X Sauerstoff ist und $R^1$ die oben angegebene Bedeutung hat.
oder
b) für den Fall daß X Schwefel ist, in Alkohol und in Gegenwart eines tertiären Amins umsetzt, zu Verbindungen der allgemeinen Formel VIII

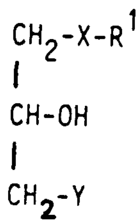

(VIII)

in welcher $R^1$ und Y die oben angegebene Bedeutung besitzen und X Schwefel ist, und diese anschließend mit einem Natriumalkoholat umsetzt zu Verbindungen der allgemeinen Formel IV, in denen X Schwefel ist,
2.) die so erhaltenen Verbindungen der allgemeinen Formel IV, in welcher $R^1$ und X die oben angegebene Bedeutung besitzen mit einem Amin der allgemeinen Formel V

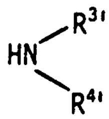

(V)

in welcher $R^{3'}$ und $R^{4'}$, die gleich oder verschieden sein können, und mit Ausnahme von Wasserstoff die Bedeutung von $R^3$ und $R^4$ haben, unter Öffnung des Epoxidrings in eine Verbindung der allgemeinen Formel VI

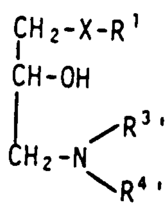

(VI)

in welcher $R^1$, $R^{3'}$, $R^{4'}$ und X die oben angegebene Bedeutung besitzen, überführt und diese
3.) in an sich bekannter Weise acyliert zu Verbindungen der allgemeinen Formel I
4.) und diese für den Fall, daß $R^3$ und/oder $R^4$ eine Benzylgruppe ist, noch katalytisch hydriert zu Verbindungen der allgemeinen Formel I, in denen $R^3$ und/oder $R^4$ Wasserstoff bedeutet.

Die für das Verfahren eingesetzten Epoxide der allgemeinen Formel II, insbesondere Epichlorhydrin und Epibromhydrin, sind käuflich, ebenso wie die Alkohole der allgemeinen Formel III und die Amine der allgemeinen Formel V.

Die Umsetzung von Verbindungen der allgemeinen Formel II zu Ethern der allgemeinen Formel IV ist nach literaturbekannten Verfahren (vgl. Synthesis 1983, 117) für Alkohole bis zu einer Kettenlänge von 10

Kohlenstoffatomen durchführbar. Längerkettige Alkohole lassen sich zur Reaktion bringen,wenn die Umsetzung in an sich bekannter Weise in einem im wesentlichen nicht mischbaren organisch/wäßrigen, stark alkalischen Zweiphasensystem das z.B. aus Dichlormethan und 50%iger Natronlauge im Verhältnis 10:1 bis 3:1 vorzugsweise jedoch 5-6:1 besteht, durchgeführt wird. Weiterhin ist es zweckmäßig bei erhöhter Temperatur, vorzugsweise beim Siedepunkt des organischen Lösungsmittels, zu arbeiten. Die Reaktionszeit beträgt 16-25h, vorzugsweise jedoch 20h. Die Reaktionsprodukte lassen sich mit bekannten Trennverfahren wie Kristallisation und/oder Chromatographie isolieren und reinigen.

Zur Herstellung der Aminoalkohole der allgemeinen Formel VI werden die Verbindungen der allgemeinen Formel IV mit einem entsprechenden Amin der allgemeinen Formel V in einem protischen Lösungsmittel, vorzugsweise Wasser, unter Erwärmen auf 40-100°C, bevorzugt 60-80°C, umgesetzt. Als weitere Lösungsmittel kommen auch niedere Alkohole, insbesondere Isopropanol, Methanol oder Ethanol, in Frage. Die Reaktionszeiten bewegen sich zwischen 1-5h, meist liegen sie bei 3h.

Die Darstellung von Thioethern der allgemeinen Formel IV erfolgt nach literaturbekanntem Verfahren (Pol.J.Chem. 58, 1237 (1984)), in dem Epoxide der allgemeinen Formel II mit Mercaptanen der allgemeinen Formel III in einem wasserfreien Alkohol wie Ethanol oder Isopropanol, bevorzugt Methanol, bei Temperaturen zwischen 20°C und 100°C, bevorzugt 40-50°C, in Gegenwart eines tertiären Amins, bevorzugt Triethylamin, unter Öffnung der Oxiranrings zu Thioethern der allgemeinen Formel VIII umgesetzt, und diese Verbindungen anschließend mit Alkoholaten wie Natriumethanolat oder Kalium-tert.-butanolat, bevorzugt Natriummethanolat, im entsprechenden Alkohol zu Verbindungen der allgemeinen Formel IV umgesetzt werden.

Die Reaktionsprodukte lassen sich mit bekannten Trennverfahren wie Kristallisation und/oder Chromatographie isolieren und reinigen.

Die Umsetzung der Aminoalkohole der allgemeinen Formel VI zu den erfindungsgemäßen Verbindungen der allgemeinen Formel I erfolgt nach an sich bekannten Verfahren entweder,
indem man (vgl. Houben-Weyl, Bd. VIII, S. 547 ff) eine äquivalante oder überschüssige, bevorzugt jedoch die doppelte Menge eines entsprechenden Carbonsäureanhydrids zusammen mit einem tert. Amin, bevorzugt wasserfreiem Pyridin, als Hilfsbase und Lösungsmittel zugleich, mit dem betreffenden Aminoalkohol im Temperaturbereich von 5-50°C, vorzugsweise jedoch bei 25°C, für 15-30h, bevorzugt für 24h, zur Reaktion bringt, oder
indem man(vgl. Houben-Weyl, Bd. VIII, S. 543 ff) eine äquivalente Menge eines entsprechenden Carbonsäurehalogenides, insbesondere eines Carbonsäurechlorides, zusammen mit der äquivalenten Menge eines tert. Amins, bevorzugt Triethylamin, in einem inerten Lösungsmittel wie Dichlormethan, 1,1,2-Trichlorethan oder auch Toluol mit dem betreffenden Aminoalkohol bei Temperaturen von 5-50°C, bevorzugt 25°C, für 1-4h, vorzugsweise jedoch für 2h, reagieren läßt. Die Isolierung und Reinigung der Produkte erfolgt für beide Varianten durch Chromatographie und/oder Kristallisation.

Die gegebenfalls erforderliche katalytische Hydrierung von N-Benzyl bzw. N,N-Dibenzylverbindungen der allgemeinen Formel I wird durchgeführt, indem man die entsprechenden Edukte in polaren Lösungsmitteln wie Methanol oder Ethanol löst und bevorzugt bei Atmosphärendruck und Raumtemperatur in Gegenwart von Pd/C als Katalysator mit Wasserstoff sättigt. Die Gewinnung der Produkte erfolgt durch Kristallisation entsprechender Salze, vorzugsweise der Hydrochloride.

Da die erfindungsgemäßen Verbindungen der allgemeinen Formel I an C-2 ein chirales Zentrum aufweisen können, liegen sie entweder als racemische Gemische oder in Form der Enantiomeren vor.

Da die Verbindungen der allgemeinen Formel I an C-3 ein basisches Zentrum besitzen, überführt man sie zum Zwecke der Reinigung und aus galenischen Gründen mit Hilfe von anorganischen und organischen Säuren bevorzugt in kristalline, pharmakologisch verträgliche Salze. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Oxalsäure oder Bernsteinsäure in Frage.

Die erfindungsgemäßen Verbindungen der allgemeinen Formel I weisen interessante pharmakologische Eigenschaften auf. Sie stellen die erste Substanzgruppe dar, welche die Proteinkinase C (PKC) hemmt, und die durch Diacylglycerole wie z.B. Oleoylacetylglycerol stimulierte Thrombozytenaggregation verhindert.

Die Proteinkinase C spielt eine herausragende Rolle bei der Regulation zellulärer Vorgänge, die eng verknüpft sind mit der physiologischen Kontrolle von kontraktilen, sekretorischen und proliferativen Prozessen (Y. Nishizuka, Nature 308, 693-698 (1984)). Die physiologische Aktivierung der Proteinkinase C erfolgt über einen, durch rezeptor-vermittelten Abbau membrangebundener Phosphatidylinositole entstandenen "second messenger", das Diacylglycerol.

Aufgrund dieser Eigenschaften können die erfindungsgemäßen Verbindungen zur Behandlung von Herz- und Gefäßkrankheiten wie Thrombosen, Arteriosklerose, Hypertension, von rheumatischen Entzündungsprozessen, Allergien, Krebs und bestimmten degenerativen Schäden des Zentralnervensystems

verwendet werden.

Die folgenden Beispiele dienen der näheren Erläuterung der Erfindung:

**Beispiel 1**

(±)-3-Dimethylamino-1-octadecyloxy-2-propylacetat • Hydrochlorid

Eine Mischung von 2.7 g (7 mmol) (±)-3-Dimethylamino-1-octadecyloxy-2-propanol und 2.2 g (21 mmol) Acetanhydrid in 20 ml Pyridin wird für 24h bei Raumtemperatur gerührt. Man destilliert das Lösungsmittel und überschüssiges Acetanhydrid im Vakuum ab und nimmt den Rückstand in 150 ml Diethylether auf. Nach Versetzen mit HCl-Gas-gesättigtem Ether wird der Niederschlag abgesaugt, mit Ether gewaschen, in 100 ml Ether suspendiert und anschließend für 1h gerührt. Nach dem Absaugen erhält man ein farbloses Produkt vom Schmp. 74-75 ° C, Ausbeute 70%.

Das als Vorstufe verwendete (±)-3-Dimethylamino-1-octadecyloxy-2-propanol wird wie folgt hergestellt: 5.1 g (16 mmol) (±)-1-Octadecyloxy-2,3-epoxypropan und 28 g einer 40%igen Lösung von Dimethylamin in Wasser werden 3h unter Rühren auf 80 ° C erwärmt. Von der abgekühlten Lösung trennt man den festen Rückstand ab, wäscht ihn mit Wasser und löst ihn in 400 ml Dichlormethan. Nach Zugabe von 100 ml 2N HCl wird 30 Minuten intensiv gerührt, die organische Phase abgetrennt, über $MgSO_4$ getrocknet und das Lösungsmittel im Vakuum entfernt. Nach Aufnahme des Rückstandes in 200 ml n-Pentan unter Rückfluß wird die heiße Suspension filtriert und der Rückstand nach Lösen in Dichlormethan mit 2N Natronlauge behandelt. Nach dem Abtrennen der organischen Phase, Trocknen über $MgSO_4$ und Abdestillieren des Lösungsmittels im Vakuum wird das Produkt vom Schmp. 72-82 ° C isoliert.

Das als Ausgangsmaterial verwendete (±)-1-Octadecyloxy-2,3-epoxypropan wird wie folgt hergestellt: Eine Mischung aus 70 ml 50%iger Natronlauge, 46.2 g (0.5 mol) Epichlorhydrin, 27.0 g (0.1 mol) Octadecanol und 1.7g Tetrabutylammoniumhydrogensulfat in 400 ml Dichlormethan wird 16h bei 40 ° C intensiv gerührt. Nach Zugabe von weiteren 1.0 g Katalysator wird noch 4h bei dieser Temperatur gerührt. Von der abgekühlten Reaktionslösung wird die organische Phase separiert, mit Wasser gewaschen, über $MgSO_4$ getrocknetund im Vakuum vom Lösungsmittel befreit.

Das restliche Epichlorhydrin wird abdestilliert und der Rückstand säulenchromatographisch aufgetrennt (Kieselgel, Dichlormethan/Cyclohexan 2:1) Farbloses Produkt vom Schmp. 74-75 ° C.

In analoger Weise werden erhalten:

(±)-1-Octadecyloxy-3-piperidino-2-propylacetat • Hydrochlorid (1.a)
Schmp. 105-106 ° C aus Ether, Ausbeute 69,9%
(±)-3-Dibenzylamino-1-octadecyloxy-2-propylacetat • Hydrochlorid (1.b)
Schmp. > 64 ° C aus n-Pentan, Ausbeute 68%
(±)-3-Dimethylamino-1-propoxy-2-propylpropionat • Oxalat (1.c)
Schmp. 110-115 ° C aus Ether, Ausbeute 62,5%
1-(2-Methyl)-piperazinyl-3-octadecyloxy-2-propylacetat • Oxalat (1.d)
Schmp. > 75 ° C aus Ethanol, Ausbeute 70%
(±)-3-Dimethylamino-1-tetradecyloxy-2-propylacetat • Hydrochlorid (1.e)
Schmp. 61-65 ° C aus n-Pentan, Ausbeute 93,8%
(±)-3-Dimethylamino-1-hexadecyloxy-2-propylacetat • Hydrochlorid (1.f)
Schmp. 48-52 ° C aus n-Pentan, Ausbeute 71%
(±)-3-Dimethylamino-1-eicosanyloxy-2-propylacetat • Hydrochlorid (1.g)
Schmp. > 77 ° C aus Ether, Ausbeute 98%
(±)-3-Di-n-butylamino-1-octadecyloxy-2-propylacetat • Oxalat (1.h)
Schmp. 70-78 ° C aus n-Pentan, Ausbeute 18,7%
(±)-3-Dimethylamino-1-octadecyloxy-2-propylisobutyrat • Hydrochlorid (1.i)
Schmp. 92-94 ° C aus n-Pentan, Ausbeute 56,2%
(±)-3-Dimethylamino-1-isobutyloxy-2-propylacetat • Oxalat (1.j)
Schmp. 126-131 ° C aus Ethylacetat/Aceton, Ausbeute 34,5%
(±)-3-(N-Benzyl-N-methyl)amino-1-octadecyloxy-2-propylacetat • Hydrochlorid (1.k)
Schmp. 81-86 ° C aus n-Pentan, Ausbeute 76,6%
(±)-3-Dimethylamino-1-(2-hexadecyloxy)-2-propylacetat • Hydrochlorid (1-l)
Schmp. 78-89 ° C aus n-Pentan, Ausbeute 83,6%
(±)-3-Dimethylamino-1-(9-cis-octadecenyloxy)-2-propylacetat • Hydrochlorid (1.m)
Ausbeute 90,5%

$^1$H-NMR ($CDCl_3$): = 5.25-5.4 (2H,m),5.05-5.15 (1H,m), 3.35-3.6 (4H,m), 2.4-2.6 (2H,m), 2.25 (6H,s),

2.05 (3H,s), 1.9-2.15 (4H,m),1.4-1.65 (3H,m), 1.15-1.4 (21H,m), 0.90 (3H,t).

**Beispiele 2**

(±)-3-Amino-1-octadecyloxy-2-propylacetat • Hydrochlorid

1.5 g (2.5 mmol) (±)-3-Dibenzylamino-1-octadecyloxy-2-propylacetat • Hydrochlorid (1.b) werden in 30 ml Ethanol gelöst und bei Atmosphärendruck und Raumtemperatur in Gegenwart von 0.3 g Pd/C als Katalysator bis zur Sättigung mit Wasserstoff umgesetzt. Man filtriert anschließend, destiliert das Lösungsmittel im Vakuum ab und nimmt den Rückstand in 40 ml Ether auf. Unter Rühren scheidet sich das Produkt vom Schmp. 62° C in fester Form ab, mit einer Ausbeute von 95%.

In gleicher Weise wird das folgende Beispiel hergestellt:
(±)-3-Methylamino-1-octadecyloxy-2-propylacetat • Hydrochlorid (2.a)
Schmp. 109-113° C aus n-Hexan, Ausbeute 61%.

**Beispiel 3**

(±)-3-Dimethylamino-1-octadecylthio-2-propylacetat Hydrochlorid

1.3 g (3.4 mmol) (±)-3-Dimethylamino-1-octadecylthio-2-propanol werden zusammen mit 0.7 g (6.7 mmol) Acetanhydrid in 30 ml wasserfreiem Pyridin gelöst und 18 h bei Raumtemperatur gerührt. Man destilliert sodann das Lösemittel im Vakuum ab und nimmt den Rückstand in 20 ml n-Hexan auf. Nach Stehenlassen bei -20° C kristallisieren 200 mg Edukt aus. Man filtriert das Edukt ab, befreit die Mutterlauge im Vakuum vom Lösemittel und nimmt den Rückstand in 60 ml Ether auf. Unter Rühren fügt man bis zur sauren Reaktion eine mit HCl-gesättigte Ether-Lösung hinzu, saugt sodann das sich gebildete Hydrochlorid ab, wäscht es mit Ether und trocknet es im Vakuum. Farblose Substanz vom Schmp. 76-80° C Ausbeute 88,2%.

Das als Vorstufe verwendete (±)-3-Dimethylamino-1-octadecylthio-2-propanol wird wie folgt hergestellt:
Ein Reaktionsgemisch aus 1.2 g (3.5 mmol) (±)-1-Octadecylthio-2,3-epoxypropan und 2.0 g (17.5 mmol) einer 40%igen Lösung von Dimethylamin in Wasser wird 4h auf 80° C erwärmt. Die abgekühlte Lösung wird in 100 ml Dichlormethan und 100 ml Wasser versetzt, die organische Phase wird abgetrennt, über Natriumsulfat getrocknet und anschließend im Vakuum vom Lösemittel befreit. Der Rückstand wird in 30 ml Ether aufgenommen und 30 MInuten auf -20° C gekühlt. Man separiert den sich gebildeten Niederschlag, befreit die Mutterlauge vom Lösemittel und isoliert so das Produkt als ein bei Raumtemperatur erstarrendes Öl.

Das als Vorstufe verwendete (±)-1-Octadecylthio-2,3-epoxypropan wird wie folgt hergestellt:
In eine Suspension aus o.58 g (18.5 mmol) Natriumhydrid (80%ig in Paraffinöl) in 50 ml wasserfreiem Tetrahydrofuran werden unter Wasserkühlung in einer Stickstoffatmosphäre langsam 20 ml wasserfreies Methanol getropft. In die klare Lösung gibt man sodann langsam eine Lösung von 7.o g (18.5 mmol) (±)-3-Chlor-1-octadecylthio-2-propanol in 30 ml wasserfreiem Methanol. Man läßt anschließend noch 1 h bei Raumtemperatur rühren, destilliert die Lösemittel im Vakuum ab und nimmt den Rückstand in 200 ml Dichlormethan auf. Man wäscht die organische Phase mit 50 ml Wasser, trocknet sie über Natriumsulfat und destilliert anschließend das Lösemittel im Vakuum ab. Farbloses Produkt vom Erwp. > 33° C.

Das als Vorstufe verwendete (±)-3-Chlor-1-octadecylthio-2-propanol wird wie folgt hergestellt:
Ein Reaktionsgemisch aus 14.3 g (50 mmol) Octadecylmercaptan, 4.6 g (50 mmol) Epichlorhydrin und 5.1 g (50 mmol) Triethylamin in 100 ml wasserfreiem Methanol wird 2 h bei 45 °C gerührt. Man kühlt sodann auf Raumtemperatur ab, saugt den schleimigen Niederschlag ab, wäscht ihn mit Methanol und trennt ihn säulenchromatographisch auf (Kieselgel, Ether/Cyclohexan = 1:3). Mit der dritten Fraktion wird das Produkt vom Erweichungspunkt > 45° C isoliert.

Die folgenden Vergleichsversuche veranschaulichen die pharmakologische Wirksamkeit der erfindungsgemäßen Verbindungen der allgemeinen Formel I:

1. Hemmung von Proteinkinase C

Zur Klärung des hemmenden Einflusses der erfindungsgemäßen Verbindungen der allgemeinen Formel I auf die Calcium- und Phospholipid-abhängige Proteinkinase (PKC) wurde diese Enzymaktivität ausgehend von einem Hühnermagenextrakt angereichert. In Anlehnung an in der Literatur beschriebene Reinigungsschritte (J. Biol. Chem. 260, 15718-15722, 1985) wurde bei der aus 2 Schritten bestehenden Reinigung die

Eigenschaft des Enzyms ausgenutzt, in Anwesenheit von Calcium an Zellmembranen zu binden und durch Calciumchelatoren (EGTA) wieder abgelöst zu werden. Beim 1. Anreicherungsschritt erfolgte die Bindung der PKC an Membranen des Hühnermagenextraktes und im 2. Schritt an sogenannte Inside / Out-Vesikel von Erythrozyten. Nach Ablösung von den Erythrozyten-Membranen und Umpufferung lag die PKC-Präparation in 10 mM HEPES, 1 mM DTT, 0,1% PEG 20000, pH 7,5 vor und konnte unter diesen Bedingungen bei -70 °C für mehrere Monate ohne Aktivitätsverlust gelagert werden.

Die Aktivität des Enzyms wurde über den Einbau von Phosphor-32-markiertem Phosphat in das durch PKC phosphorylierbare Protein Histon H-1 bestimmt. Der Test enthält dabei folgende Komponenten: 50 mM HEPES (pH 7,5), 1 mM DTT, 5 mM $MgCl_2$, ca. 10 µM freies $Ca^{2+}$, 200 µg/ml Histon, 5 µg/ml Phosphatidylserin, 1 µg/ml 1,2-Diolein, 10µM ATP, eine jeweils geeignete Menge der PKC-Präparation und gegebenenfalls eine Testsubstanz. In Abwesenheit der beiden PKC-Aktivatoren Phosphatidylserin und 1,2-Diolein war ebenfalls ein geringer Einbau von radioaktivem Phosphat meßbar; dieses Verhalten wurde als PKC-unabhängige Kinaseaktivität interpretiert. Die unter Standardbedingungen gemessene Kinase-Aktivität in Anwesenheit bzw. Abwesenheit von PKC-Aktivatoren (und ohne Testsubstanz) wurde jeweils als 100% gesetzt und die Hemmwirkung der Verbindungen der allgemeinen Formel I prozentual darauf bezogen.

Die Ergebnisse sind in Tabelle 1 dargestellt.

TABELLE 1

| Inhibierung von PS/DO vermittelter Protein Kinase C - Aktivität und von OAG-induzierter Plättchen-Aggregation | | | |
|---|---|---|---|
| Beispiel | Konzentration (mol/l) | Inhibierung der PS/DO vermittelten PKC-Aktivität (%) | Inhibierung der OAG induzierten Plättchen Aggregation (mol/1) |
| 1 | $10^{-4}$ | 86 | 100 |
| 1 | $10^{-5}$ | 61 | 48 |
| 1 | $10^{-6}$ | 24 | |
| 1.a | $10^{-4}$ | 25 | 54 |
| 1.b | $10^{-5}$ | 22 | |
| 1.c | $10^{-4}$ | 9 | |
| 1.d | $10^{-5}$ | 61 | 100 |
| 1.d | $3x10^{-6}$ | | 14 |
| 1.d | $10^{-6}$ | 18 | |
| 1.e | $10^{-5}$ | 14 | |
| 1.f | $10^{-5}$ | 27 | |
| 1.g | $10^{-5}$ | 39 | 53 |
| 1.l | $10^{-5}$ | 31 | |
| 1.m | $10^{-5}$ | 48 | |
| 1.m | $10^{-6}$ | 14 | |
| 2 | $10^{-4}$ | 44 | |
| 2 | $10^{-5}$ | 18 | |
| 2.a | $10^{-5}$ | 35 | |
| 2.a | $10^{-6}$ | 13 | |
| 3 | $10^{-4}$ | 70 | |
| 3 | $10^{-5}$ | 22 | |
| PS = Phosphatidylserin DO = 1,2-Diolein<br>OAG = Oleoylacetylglycerol | | | |

2. Einfluß von Beispiel 1 auf die Oleoylacetylglycerol-stimulierte Plättchenaggregation

Für die Durchführung der Aggregationsexperimente wurden gelfiltrierte Human-Thrombozyten verwendet. Diese wurden folgenderweise bereitgestellt: Nach Zentrifugation von Humanblut für 15 Minuten bei 60xg wurde der Überstand, das plättchenreiche Plasma, für die Gelfiltration verwendet. Durch die Gelfiltration mittels einer mit Sepharose CL-2B gepackten Säule wurden die Plättchen in ein calciumfreies,

definiertes Puffermilieu (15 mM Tris, 150 mM NaCl, 5,5 mM Glucose, 0,2% Rinderserum-Albumin, pH 7,4) überführt. Anschließend wurden die Plättchen auf eine Konzentration von $10^{-4}$ M Calcium recalcifiziert. Die Plättchen wurden dann bei 37 °C in einem Aggregometer inkubiert und nach Auslösung der Aggregation durch einen Stimulus die Abnahme der Absorption als Maß für die Aggregation bestimmt. Als aggregationsauslösender Stimulus wurde 1-Oleoyl-2-acetylglycerol(OAG), ein Aktivator des Enzyms Proteinkinase C (J. Biol. Chem. 258, 6701-6704, 1983) in einer Konzentration von 5 x $10^{-5}$ M verwendet. Die unter diesen Bedingungen erzielte Aggregation wurde als 100%-Kontrolle betrachtet. Die Durchführung der Experimente zum Einfluß von Beispiel 1 auf die OAG-stimulierte Aggregation erfolgte derart, daß die Plättchen zuerst 3 Minuten mit der jeweiligen Konzentration an Beispiel 1 inkubiert wurden und anschließend mit OAG die Aggregation ausgelöst wurde. Das reduzierte Ausmaß der Aggregation wurde jeweils als Prozentsatz der 100%-Kontrolle berechnet.

Die Ergebnisse sind ebenfalls in Tabelle 1 dargestellt.

**Patentansprüche**

**Patentansprüche für folgende Vertragsstaaten : BE, CH, DE, FR, GB, IT, LI, LU, NL, SE**

**1.** 2-Acyloxypropylamin-Derivate der allgemeinen Formel I

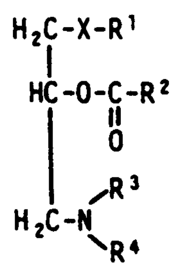

(I)

in welcher

$R^1$ eine geradkettige oder verzweigte, gesättigte oder ungesättigte Alkylgruppe mit bis zu 20 Kohlenstoffatomen, darstellt,

$R^2$ eine geradkettige oder verzweigte Alkylgruppe mit bis zu 4 Kohlenstoffatomen bedeutet,

$R^3$ und $R^4$ die gleich oder verschieden sein können, Wasserstoff oder eine gegebenenfalls phenylsubstituierte geradkettige oder verzweigte Alkylgruppe mit bis zu 14 Kohlenstoffatomen oder gemeinsam einen unsubstituierten oder $C_1$ bis $C_4$ alkylsubstituierten Piperidin-oder Piperazinring bilden und

X Sauerstoff oder Schwefel bedeuten

mit Ausnahme von 3-Diethylamino-1-hexyloxy-2-propylacetat,
sowie deren pharmakologisch unbedenkliche Salze.

**2.** 2-Acyloxypropylamin-Derivate gemäß Anspruch 1,
in welcher
$R^1$ eine Propyl-, Octadecyl-, Octadecenyl-, Tetradecyl-, Hexadecyl-, Eicosanoyl- oder Isobutylgruppe,
$R^2$ eine Methyl- oder Isopropylgruppe,
$R^3$ und $R^4$, die gleich oder verschieden sein können, Wasserstoff, Methyl-, Ethyl-, Propyl- oder Benzylgruppen oder gemeinsam eine Piperidin- oder 2-Methylpiperazingruppe und
X Sauerstoff oder Schwefel bedeuten.

**3.** (±)-3-Dimethylamino-1-octadecyloxy-2-propylacetat.

**4.** 1-(2-Methyl)-piperazinyl-3-octadecyloxy-2-propylacetat • Oxalat

**5.** (±)-3-Dimethylamino-1-(9-cis-octadecenyloxy)-2-propylacetat • Hydrochlorid

**6.** Verfahren zur Herstellung von 2-Acyloxypropylamin-Derivaten gemäß Anspruch 1, dadurch gekennzeichnet, daß man

A) in an sich bekannter Weise eine Verbindung der allgemeinen Formel II

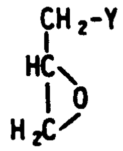

(II)

in welcher Y eine gute Abgangsgruppe, wie z.B. eine Tosylgruppe oder ein Halogenatom, bedeutet, mit einer Verbindung der allgemeinen Formel III,

$HX-R^1$ (III)

in der $R^1$ und X die oben angegebene Bedeutung haben, entweder

a) für den Fall, daß X Sauerstoff ist, in einem organisch/wäßrigen Zweiphasensystem umsetzt zu Verbindungen der allgemeinen Formel IV

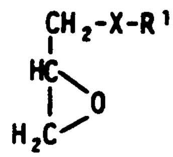

(IV)

in denen X Sauerstoff ist und $R^1$ die oben angegebene Bedeutung hat.
oder

b) für den Fall daß X Schwefel ist, in Alkohol und in Gegenwart eines tertiären Amins umsetzt, zu Verbindungen der allgemeinen Formel VIII

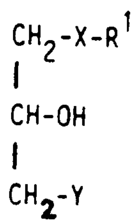

(VIII)

in welcher $R^1$ und Y die oben angegebene Bedeutung besitzen und X Schwefel ist, und diese anschließend mit einem Natriumalkoholat umsetzt zu Verbindungen der allgemeinen Formel IV, in denen X Schwefel ist,

B) die so erhaltenen Verbindungen der allgemeinen Formel IV, in welcher $R^1$ und X die oben angegebene Bedeutung besitzen, mit einem Amin der allgemeinen Formel V

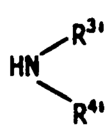

(V)

in welcher $R^{3'}$ und $R^{4'}$, die gleich oder verschieden sein können, und mit Ausnahme von Wasserstoff die Bedeutung von $R^3$ und $R^4$ haben, unter Öffnung des Epoxidrings in eine Verbindung der allgemeinen Formel VI

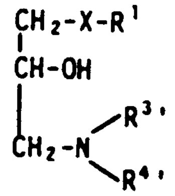

(VI)

in welcher $R^1$, $R^{3'}$, $R^{4'}$ und X die oben angegebene Bedeutung besitzen, überführt und diese
C) in an sich bekannter Weise acyliert zu Verbindungen der allgemeinen Formel I
D) und diese für den Fall, daß $R^3$ und/oder $R^4$ eine Benzylgruppe ist, noch katalytisch hydriert zu Verbindungen der allgemeinen Formel I, in denen $R^3$ und/oder $R^4$ Wasserstoff bedeutet.

**7.** Arzneimittel enthaltend mindestens ein 2-Acyloxypropylamin-Derivat gemäß den Ansprüchen 1 bis 5 in Verbindung mit üblichen Hilfs- und Zusatzstoffen.

**8.** Verwendung von 2-Acyloxypropylamin-Derivaten gemäß den Ansprüchen 1 bis 5 zur Herstellung von Arzneimitteln für die Inhibierung der Proteinkinase C und die Behandlung von Herz-Kreislauf-Erkrankungen wie Thrombosen, Arteriosklerosen, Hypertension, rheumatischen und inflammatorischen Erkrankungen, Allergien, Tumoren und gewissen degenerativen Schädigungen des Zentralnervensystems.

**Patentansprüche für folgende Vertragsstaaten : AT, ES, GR**

**1.** Verfahren zur Herstellung von 2-Acyloxypropylamin-Derivaten der allgemeinen Formel I

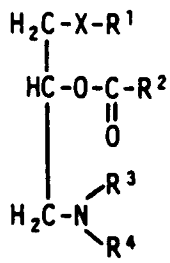

(I)

in welcher

| | |
|---|---|
| $R^1$ | eine geradkettige oder verzweigte, gesättigte oder ungesättigte Alkylgruppe mit bis zu 20 Kohlenstoffatomen, darstellt, |
| $R^2$ | eine geradkettige oder verzweigte Alkylgruppe mit bis zu 4 Kohlenstoffatomen bedeutet, |
| $R^3$ und $R^4$ | die gleich oder verschieden sein können, Wasserstoff oder eine gegebenenfalls phenylsubstituierte geradkettige oder verzweigte Alkylgruppe mit bis zu 14 Kohlenstoffatomen oder gemeinsam einen unsubstituierten oder $C_1$ bis $C_4$ alkylsubstituierten Piperidin-oder Piperazinring bilden und |
| X | Sauerstoff oder Schwefel bedeuten, |

mit Ausnahme von 3-Diethylamino-1-hexyloxy-2-propylacetat
sowie deren pharmakologisch unbedenkliche Salze, dadurch gekennzeichnet, daß man
A) in an sich bekannter Weise eine Verbindung der allgemeinen Formel II

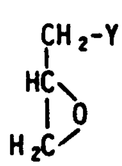

(II)

in welcher Y eine gute Abgangsgruppe, wie z.B. eine Tosylgruppe oder ein Halogenatom, bedeutet, mit einer Verbindung der allgemeinen Formel III,

$HX\text{-}R^1$ (III)

in der $R^1$ und X die oben angegebene Bedeutung haben, entweder

a) für den Fall, daß X Sauerstoff ist, in einem organisch/wäßrigen Zweiphäsensystem umsetzt zu Verbindungen dar allgemeinen Formel IV

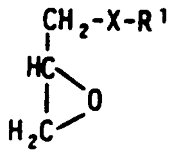

(IV)

in denen X Sauerstoff ist und $R^1$ die oben angegebene Bedeutung hat

oder

b) für den Fall daß X Schwefel ist, in Alkohol und in Gegenwart eines tertiären Amins umsetzt, zu Verbindungen der allgemeinen Formel VIII

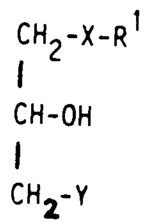

(VIII)

in welcher $R^1$ und Y die oben angegebene Bedeutung besitzen und X Schwefel ist, und diese anschließend mit einem Natriumalkoholat umsetzt zu Verbindungen der allgemeinen Formel IV, in denen X Schwefel ist,

B) die so erhaltenen Verbindungen der allgemeinen Formel IV, in welcher $R^1$ und X die oben angegebene Bedeutung besitzen, mit einem Amin der allgemeinen Formel V

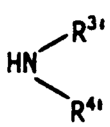

(V)

in welcher $R^{3'}$ und $R^{4'}$, die gleich oder verschieden sein können, und mit Ausnahme von Wasserstoff die Bedeutung von $R^3$ und $R^4$ haben, unter Öffnung des Epoxidrings in eine Verbindung der allgemeinen Formel VI

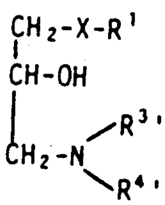

(VI)

in welcher $R^1$, $R^{3'}$, $R^{4'}$ und X die oben angegebene Bedeutung besitzen, überführt und diese

C) in an sich bekannter Weise acyliert zu Verbindungen der allgemeinen Formel I

D) und diese für den Fall, daß $R^3$ und/oder $R^4$ eine Benzylgruppe ist, noch katalytisch hydriert zu Verbindungen der allgemeinen Formel I, in denen $R^3$ und/oder $R^4$ Wasserstoff bedeutet.

**2.** Verfahren gemäß Anspruch 1 zur Herstellung von 2-Acyloxypropylamin-Derivaten der allgemeinen Formel I, in welcher
$R^1$ eine Propyl-, Octadecyl-, Octadecenyl-, Tetradecyl-, Hexadecyl-, Eicosanoyl- oder Isobutylgruppe,
$R^2$ eine Methyl- oder Isopropylgruppe,
$R^3$ und $R^4$, die gleich oder verschieden sein können, Wasserstoff, Methyl-, Ethyl-, Propyl- oder Benzylgruppen oder gemeinsam eine Piperidin- oder 2-Methylpiperazingruppe und
X Sauerstoff oder Schwefel bedeuten.

**3.** Verfahren gemäß Anspruch 1 zur Herstellung von (±)-3-Dimethylamino-1-octadecyloxy-2-propylacetat,

**4.** Verfahren gemäß Anspruch 1 zur Herstellung von 1-(2-Methyl)-piperazinyl-3-octadecyloxy-2-propylacetat • Oxalat

**5.** Verfahren gemäß Anspruch 1 zur Herstellung von (±)-3-Dimethylamino-1-(9-cis-octadecenyloxy)-2-propylacetat • Hydrochlorid

**6.** Verwendung von 2-Acyloxypropylamin-Derivaten gemäß den Ansprüchen 1 bis 5 zur Herstellung von Arzneimitteln für die Inhibierung der Proteinkinase C und die Behandlung von Herz-Kreislauf-Erkrankungen wie Thrombosen, Arteriosklerosen, Hypertension, rheumatischen und inflammatorischen Erkrankungen, Allergien, Tumoren und gewissen degenerativen Schädigungen des Zentralnervensystems.

## Claims

**Claims for the following Contracting States : BE, CH, DE, FR, GB, IT, LI, LU, NL, SE**

**1.** 2-Acyloxypropylamine derivatives of the general formula I

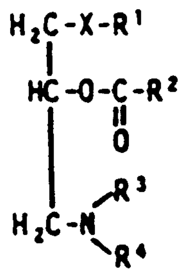

(I)

in which $R^1$ represents a straight-chained or branched, saturated or unsaturated alkyl group with up to 20 carbon atoms, $R^2$ signifies a straight-chained or branched alkyl group with up to 4 carbon atoms, $R^3$ and $R^4$, which can be the same or different, signify hydrogen or a possibly phenyl-substituted straight-chained or branched alkyl group with up to 14 carbon atoms or together form an unsubstituted or $C_1$ to $C_4$ alkyl-substituted piperidine or piperazine ring and X signifies oxygen or sulphur, with the exception of 3-diethylamino-1-hexyloxy-2-propyl acetate, as well as their pharmacologically acceptable salts.

**2.** 2-Acyloxypropylamine derivatives according to claim 1, in which $R^1$ signifies a propyl-, octadecyl-, octadecenyl-, tetradecyl, hexadecyl, eicosanoyl- or isobutyl group, $R^2$ signifies a methyl or isopropyl group. $R^3$ and $R^4$, which can be the same or different, signify hydrogen, methyl-, ethyl-, propyl- or benzyl groups or together a piperidine or 2-methyl piperazine ring group and X signifies oxygen or sulphur.

**3.** (±)-3-dimethylamino-1-octadecyloxy-2-propyl acetate.

**4.** 1-(2-methyl)-piperazinyl-3-octadecyloxy-2-propyl acetate oxalate.

**5.** (±)-3-dimethylamino-1-(9-cis-octadecenyloxy)-2-propyl acetate hydrochloride.

**6.** Method for the preparation of 2-acyloxypropylamine derivatives according to claim 1, characterised in that,

A) in a manner known per se, a compound of the general formula II

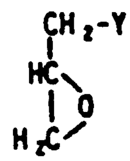

(II)

in which Y is a good leaving group, such as e.g. a tosyl group or a halogen atom, with a compound of the general formula III

$HX\text{-}R^1$ (III)

in which $R^1$ and X have the above-given meaning, either

a) for the case where X is oxygen, in an organic/aqueous two-phase system, is converted to compounds of the general formula IV

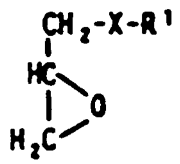

(IV)

in which X is oxygen and $R^1$ has the above-given meaning.

or

b) for the case where X is sulphur, is converted in alcohol and in the presence of a tertiary amine, to compounds of the general formula VIII

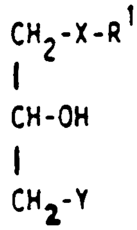

(VIII)

in which $R^1$ and Y have the above-given meaning and X is sulphur, and then with a sodium alcoholate this is converted to compounds of the general formula IV, in which X is sulphur.

B) the compounds of the general formula IV thus obtained, in which $R^1$ and X have the above-given meaning, with an amine of the general formula V

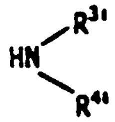

(V)

in which $R^{3'}$ and $R^{4'}$ which can be the same or different have the meaning of $R^3$ and $R^4$ with the exception of hydrogen, with opening of the epoxide ring, are converted into a compound of the general formula VI

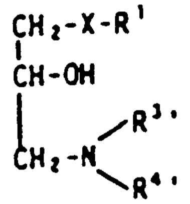

(VI)

in which $R^1$, $R^{3'}$, $R^{4'}$ and X have the above-given meaning

C) and this is acylated in a manner known per se to compounds of the general formula I.

D) and in the case where $R^3$ and/or $R^4$ is/are a benzyl group, this is then catalytically hydrogenated to compounds of the general formula I in which $R^3$ and/or $R^4$ signify hydrogen.

**7.** A medicine, containing at least one 2-acyloxypropylamine derivative according to one of claims 1 to 5 combined with the usual auxiliary and added substances.

**8.** Use of 2-acyloxyproplyamine derivatives according to claims 1 to 5 for manufacturing medicines for the inhibition of protein kinase C and the treatment of heart-circulation diseases such as thromboses, arterioscleroses, hypertension, rheumatic and inflammatory diseases, allergies, cancers and certain degenerative damages of the central nervous system.

**Claims for the following Contracting States : AT, ES, GR**

**1.** A method for producing 2-acyloxypropylamine derivatives of the general formula I

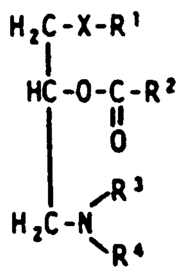

(I)

in which $R^1$ represents a straight-chained or branched, saturated or unsaturated alkyl group with up to 20 carbon atoms, $R^2$ signifies a straight-chained or branched alkyl group with up to 4 carbon atoms, $R^3$ and $R^4$, which can be the same or different, signify hydrogen or a possibly phenyl-substituted straight-chained or branched alkyl group with up to 14 carbon atoms or together form an unsubstituted or $C_1$ to $C_4$ alkyl-substituted piperidine or piperazine ring and X signifies oxygen or sulphur, with the exception of 3-diethylamino-1-hexyloxy-2-propyl acetate, as well as their pharmacologically acceptable salts characterised in that

A) in a manner known per se, a compound of the general formula II

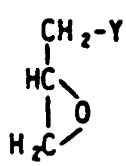

(II)

in which Y is a good leaving group, such as e.g. a tosyl group or a halogen atom, with a compound of the general formula III

$HX-R^1$ (III)

in which $R^1$ and X have the above-given meaning, either

a) for the case where X is oxygen, in an organic/aqueous two-phase system, is converted to compounds of the general formula IV

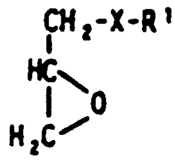

(IV)

in which X is oxygen and $R^1$ has the above-given meaning,

or

b) for the case where X is sulphur, is converted in alcohol and in the presence of a tertiary amine, to compounds of the general formula VIII

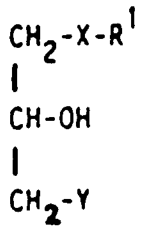

(VIII)

in which $R^1$ and Y have the above-given meaning and X is sulphur, and then with a sodium alcoholate this is converted to compounds of the general formula IV, in which X is sulphur.

B) the compounds of the general formula IV thus obtained, in which $R^1$ and X have the above-given meaning, with an amine of the general formula V

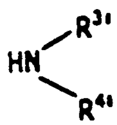

(V)

in which $R^{3'}$ and $R^{4'}$ which can be the same or different have the meaning of $R^3$ and $R^4$ with the exception of hydrogen, with opening of the epoxide ring, are converted into a compound of the general formula VI

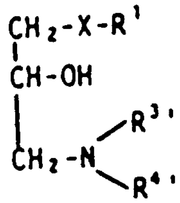

(VI)

in which $R^1$, $R^{3'}$, $R^{4'}$ and X have the above-given meaning

C) and this is acylated in a manner known per se to compounds of the general formula I.

D) and in the case where $R^3$ and/or $R^4$ is/are a benzyl group, this is then catalytically hydrogenated to compounds of the general formula I in which $R^3$ and/or $R^4$ signify hydrogen.

**2.** A method according to claim 1 for producing 2-acyloxypropylamine derivatives of the general formula I,

in which $R^1$ signifies a propyl-, octadecyl-, octadecenyl-, tetradecyl, hexadecyl, eicosanoyl- or isobutyl group, $R^2$ signifies a methyl or isopropyl group, $R^3$ and $R^4$, which can be the same or different, signify hydrogen, methyl-, ethyl-, propyl- or benzyl groups or together a piperidine or 2-methyl piperazine ring group and X signifies oxygen or sulphur.

**3.** A method according to claim 1 for producing (±)-3-dimethylamino-1-octadecyloxy-2-propyl acetate.

**4.** A method according to claim 1 for producing 1-(2-methyl)-piperazinyl-3-octadecyloxy-2-propyl acetate oxalate.

**5.** A method according to claim 1 for producing (±)-3-dimethylamino-1-(9-cis-octadecenyloxy)-2-propyl acetate hydrochloride.

**6.** Use of 2-acyloxyproplyamine derivatives according to claims 1 to 5 for producing medicines for the inhibition of protein kinase C and the treatment of heart-circulation diseases such as thromboses, arterioscleroses, hypertension, rheumatic and inflammatory diseases, allergies, cancers and certain degenerative damages of the central nervous system.

**Revendications**

**Revendications pour les Etats contractants suivants : BE, CH, DE, FR, GB, IT, LI, LU, NL, SE**

**1.** Dérivés de 2-acyloxypropylamine de formule générale I:

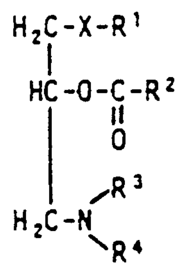

(I)

dans laquelle

| | |
|---|---|
| $R^1$ | représente un groupe alkyle saturé ou insaturé, à chaîne droite ou ramifiée, renfermant jusqu'à 20 atomes de carbone, |
| $R^2$ | signifie un groupe alkyle à chaîne droite ou ramifiée renfermant jusqu'à 4 atomes de carbone, |
| $R^3$ et $R^4$ | qui peuvent être identiques ou différents représentent un atome d'hydrogène ou un groupe alkyle à chaîne droite ou ramifiée éventuellement substitué par un radical phényle et renfermant jusqu'à 14 atomes de carbone, ou ils forment ensemble un cycle piperidine ou piperazine non substitué ou substitué par un radical alkyle en $C_1$ à $C_4$, et |
| X | signifie un atome d'oxygène ou un atome de soufre, |

à l'exception du 3-diéthylamino-1-hexyloxy-2-propylacétate ainsi que leurs sels pharmaceutiquement compatibles.

**2.** Dérivés de 2-acyloxypropylamine selon la revendication 1, caractérisés en ce que :

| | |
|---|---|
| $R^1$ | représente un groupe propyle, octadécyle, octadécényle, tétradécyle, hexadécyle, eicosanoyle ou isobutyle, |
| $R^2$ | un groupe méthyle ou isopropyle, |
| $R^3$ et $R^4$ | qui peuvent être identiques ou différents représentent un atome d'hydrogène, des groupes méthyle, éthyle, propyle ou benzyle ou ils forment ensemble un groupe piperidine ou 2-méthylpiperazine, et |
| X | représente un atome d'oxygène ou un atome de soufre. |

**3.** (±)-3-diméthylamino-1-octadécyloxy-2-propylacétate.

**4.** Oxalate de 1-(2-méthyl)-piperazinyl-3-octadécyloxy-2-propylacétate.

**5.** Chlorhydrate de (±)-3-diméthylamino-1-(9-cis-octadécényloxy)-2-propylacétate.

**6.** Procédé de préparation de dérivés de 2-acyloxypropylamine selon la revendication 1, caractérisé en ce que:

A) d'une façon connue en soi, on fait réagir un dérivé de formule générale II:

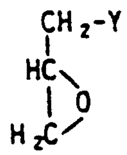

(II)

dans laquelle Y représente un groupe de départ convenable comme par exemple un groupe tosyle ou un atome d'halogène avec un dérivé de formule générale III,

$HX-R^1$ (III)

dans laquelle $R^1$ et X présentent la signification 1 indiquée ci-dessus, cette réaction soit:

a) dans le cas où X représente un atome d'oxygène, ayant lieu dans un système diphasique organique/aqueux et conduisant à des dérivés de formule générale IV:

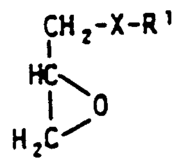

(IV)

dans laquelle X représente un atome d'oxygène et $R^1$ présente la signification indiquée ci-dessus, ou bien soit:

b) dans le cas où X représente un atome de soufre, ayant lieu dans de l'alcool et en présence d'une amine tertiaire et conduisant à des dérivés de formule générale VIII:

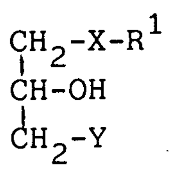

(VIII)

dans laquelle $R^1$ et Y présentent la signification indiquée ci-dessus et X représente un atome de soufre, et que l'on fait réagir ensuite avec un alcoolate de sodium pour obtenir des dérivés de formule générale IV dans lesquels X représente un atome de soufre,

B) On fait réagir les dérivés ainsi obtenus de formule générale IV, dans lesquels $R^1$ et X présentent les significations indiquées ci-dessus avec une amine de formule générale V:

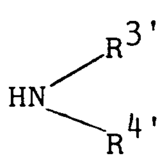

(V)

dans laquelle $R^{3'}$ et $R^{4'}$, qui peuvent être identiques ou différents et, présentent la signification de $R^3$ et $R^4$ à l'exception de l'hydrogène, avec ouverture du cycle époxyde, en un dérivé de formule générale VI:

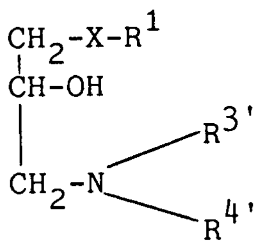

(VI)

dans laquelle $R^1$, $R^{3'}$, $R^{4'}$ et X présentent la signification indiquée ci-dessus, et

C) On procède d'une façon connue en soi à l'acylation de ces dérivés en dérivés de formule générale I,

D) et pour le cas où $R^3$ et/ou $R^4$ signifient un groupe benzyle, on procède encore à une hydrogénation catalytique de ces dérivés pour obtenir des dérivés de formule générale I, dans lesquels $R^3$ et/ou $R^4$ signifient un atome d'hydrogène.

**7.** Médicament contenant au moins un dérivé de 2-acyloxypropylamine selon l'une des revendications 1 à 5, associé à des adjuvants et additifs usuels.

**8.** Utilisation de dérivés de 2-acyloxypropylamine selon l'une des revendications 1 à 5 pour la préparation de médicaments destinés à l'inhibition de la protéine kinase C et au traitement de maladies cardiaques et circulatoires telles que thrombose, artériosclérose, hypertension, maladies rhumatismales et inflammatoires, allergies, tumeurs et certaines altérations dégénératives du système nerveux central.

**Revendications pour les Etats contractants suivants : AT, ES, GR**

**1.** Procédé de préparation de dérivés de 2-acyloxypropylamine de formule générale I:

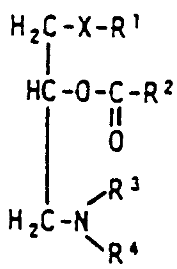

(I)

dans laquelle

$R^1$ représente un groupe alkyle saturé ou insaturé, à chaîne droite ou ramifiée, renfermant jusqu'à 20 atomes de carbone,

$R^2$ signifie un groupe alkyle à chaîne droite ou ramifiée renfermant jusqu'à 4 atomes de carbone,

$R^3$ et $R^4$ qui peuvent être identiques ou différents représentent un atome d'hydrogène ou un groupe alkyle à chaîne droite ou ramifiée éventuellement substitué par un radical phényle et renfermant jusqu'à 14 atomes de carbone, ou ils forment ensemble un cycle piperidine ou piperazine non substitué ou substitué par un radical alkyle en $C_1$ à $C_4$, et

X signifie un atome d'oxygène ou un atome de soufre,

à l'exception du 3-diéthylamino-1-hexyloxy-2-propylacétate ainsi que leurs sels pharmaceutiquement compatibles, caractérisé en ce que:

A) d'une façon connue en soi, on fait réagir un dérivé de formule générale II:

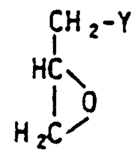

(II)

dans laquelle Y représente un groupe de départ convenable comme par exemple un groupe tosyle ou un atome d'halogène avec un dérivé de formule générale III,

$HX-R^1$ (III)

dans laquelle $R^1$ et X présentent la signification 1 indiquée ci-dessus, cette réaction soit:

a) dans le cas où X représente un atome d'oxygène, ayant lieu dans un système diphasique organique/aqueux et conduisant à des dérivés de formule générale IV:

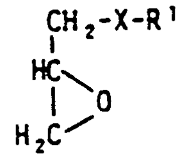

(IV)

dans laquelle X représente un atome d'oxygène et $R^1$ présente la signification indiquée ci-dessus, ou bien soit:

b) dans le cas où X représente un atome de soufre, ayant lieu dans de l'alcool et en présence d'une amine tertiaire et conduisant à des dérivés de formule générale VIII:

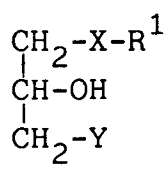

(VIII)

dans laquelle $R^1$ et Y présentent la signification indiquée ci-dessus et X représente un atome de soufre, et que l'on fait réagir ensuite avec un alcoolate de sodium pour obtenir des dérivés de formule générale IV dans lesquels X représente un atome de soufre,

B) On fait réagir les dérivés ainsi obtenus de formule générale IV, dans lesquels $R^1$ et X présentent les significations indiquées ci-dessus avec une amine de formule générale V:

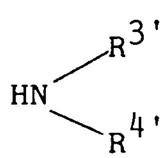

(V)

dans laquelle $R^{3'}$ et $R^{4'}$, qui peuvent être identiques ou différents et, présentent la signification de $R^3$ et $R^4$ à l'exception de l'hydrogène, avec ouverture du cycle époxyde, en un dérivé de formule générale VI:

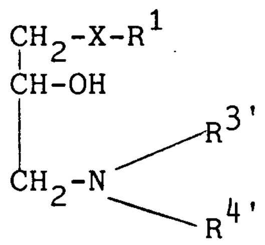

(VI)

dans laquelle $R^1$, $R^{3'}$, $R^{4'}$ et X présentent la signification indiquée ci-dessus, et

C) On procède d'une façon connue en soi à l'acylation de ces dérivés en dérivés de formule générale I,

D) et pour le cas où $R^3$ et/ou $R^4$ signifient un groupe benzyle, on procède encore à une hydrogénation catalytique de ces dérivés pour obtenir des dérivés de formule générale I, dans lesquels $R^3$ et/ou $R^4$ signifient un atome d'hydrogène.

**2.** Procédé selon la revendication 1 de préparation de dérivés de 2-acyloxypropylamine de formule générale I dans lesquels :

| | |
|---|---|
| $R^1$ | représente un groupe propyle, octadécyle, octadécényle, tétradécyle, hexadécyle, eicosanoyle ou isobutyle, |
| $R^2$ | représente un groupe méthyle ou un groupe isopropyle, |
| $R^3$ et $R^4$ | qui peuvent être identiques ou différents représentent un atome d'hydrogène, des groupes méthyle, éthyle, propyle ou benzyle ou ils forment ensemble un groupe piperidine ou 2-méthylpiperazine, et |
| X | représente un atome d'oxygène ou un atome de soufre. |

**3.** Procédé selon la revendication 1 de préparation de (±)-3-diméthylamino-1-octadécyloxy-2-propylacétate.

**4.** Procédé selon la revendication 1 de préparation d'oxalate de 1-(2-méthyl)-piperazinyl-3-octadécyloxy-2-propylacétate.

**5.** Procédé selon la revendication 1 de préparation de chlorhydrate de (±)-3-diméthylamino-1-(9-cis-octadécényloxy)- 2-propylacétate.

**6.** Utilisation de dérivés de 2-acyloxypropylamine selon l'une des revendications 1 à 5 pour la préparation de médicaments destinés à l'inhibition de la protéine kinase C et au traitement de maladies cardiaques et circulatoires telles que thrombose, artériosclérose, hypertension, maladies rhumatismales et inflammatoires, allergies, tumeurs et certains altérations dégénératives du système nerveux central.